Europäisches Patentamt

European Patent Office

Office européen des brevets

(11) Publication number: **0 309 145**
**A1**

# EUROPEAN PATENT APPLICATION

(21) Application number: **88308499.8**

(22) Date of filing: **14.09.88**

(51) Int. Cl.⁴ **C12N 1/20** , **A01N 63/00**

The applicant has filed a statement in accordance with Rule 28 (4) EPC (issue of a sample only to an expert). Accession number of the deposit: NRRL B - 18251.

(30) Priority: **22.09.87 US 99570**

(43) Date of publication of application:
**29.03.89 Bulletin 89/13**

(84) Designated Contracting States:
**AT BE CH DE ES FR GB GR IT LI LU NL SE**

(71) Applicant: **Mycogen Corporation**
**5451 Oberlin Drive**
**San Diego, CA 92121(US)**

(72) Inventor: **Nette, Kathryn M.**
**10262 Pinecastle Street**
**San Diego California 92131(US)**

(74) Representative: **Perry, Robert Edward et al**
**GILL JENNINGS & EVERY 53-64 Chancery Lane**
**London WC2A 1HN(GB)**

(54) **Bacteriophage-resistant strain of Bacillus Thuringiensis Var. San Diego.**

(57) Novel bacteriophage-resistant strains of Bacillus thuringiensis have activity against Coleoptera, e.g. strain MT-38 of the known microbe Bacillus thuringiensis var. san diego (B.t.sd.). The parent B.t.sd. and B.T.Sd. strain MT-38 have the same spectrum of activity against a number of coleopteran pests. B.t.sd. is phage-sensitive, whereas B.t.sd. strain MT-38 is phage-resistant. This difference makes B.T.Sd. strain MT-38 the preferred microbe for the preparation of pesticidal preparations active against Coleoptera.

EP 0 309 145 A1

# BACTERIOPHAGE-RESISTANT STRAIN OF BACILLUS THURINGIENSIS VAR. SAN DIEGO

## Background of the Invention

The known bacterium Bacillus thuringiensis var. san diego (B.t.sd.), NRRL B-15939, has activity against a number of insects of the order Coleoptera. Insects of the orders Lepidoptera or Diptera are not susceptible to the toxin produced by B.t.sd. (See Herrnstadt. C. et al. "A New Strain of Bacillus thuringiensis with activity against Coleopteran Insects," Bio Technology. 4:305-308.) Thus, B.t.sd. is useful to control various coleopteran pests which are responsible for significant economic losses in agriculture. Perhaps the most important coleopteran pest controllable by B.t.sd. is the Colorado potato beetle.

The utility of B.t.sd. to control coleopteran pests is somewhat dependent upon efficient and economical production of B.t.sd. cells suitable for pesticidal use. The process involves the use of fermentation means well known in the Bacillus thuringiensis art. In a standard B.t. fermentation, the harvesting of the spores and crystals is done after lysis of the B.t. cells occurs. The goal is to have as high a titer of B.t. toxin produced as possible before such lysis occurs.

It has been discovered that, unfortunately, B.t.sd., though capable of producing a toxin active against coleopteran beetles. is subject to lysing in the fermentation cycle before optimum titers of toxin have been produced. This premature lysing results in a reduced toxin titer in the fermentation beer and a resultant inferior B.t. product.

Another disadvantage of premature lysis of the B.t. cells is from the standpoint of use of treated, substantially intact B.t. cells as pesticides. In this type of process. substantially intact cells are treated with chemical or physical means to stabilize the cells and prolong the pesticidal (toxin) activity in the environment. Such a treatment of the B.t. cells is carried out after a sufficient titer of intracellular toxin has been produced by the B.t. microbe and while the microbe is still in the substantially intact form. When the B.t. cells lyse prematurely, the benefits of the encapsulation process are not realized. Accordingly, it is desirable to delay the cell lysis to allow recovery of substantially intact cells having maximum titers of intracellular toxin.

## Brief Summary of the Invention

This invention solves the problem of premature lysing of B.t.sd. cells during the fermentation cycle. More specifically, the invention concerns a novel bacteriophage (phage)-resistant strain of B.t.sd., referred to as B.t.sd. strain MT-38, which, advantageously. does not prematurely lyse during the fermentation cycle. This novel phage-resistant strain of B.t.sd. retains the toxin production characteristics of the parent B.t.sd.

B.t.sd. strain MT-38 was prepared by selection of resistant colonies from an agar plate that was covered with a lawn of B.t.sd. that was heavily contaminated with a bacteriophage. Details are given in Example 1.

A subculture of B.t.sd. strain MT-38 has been deposited in the permanent collection of the Agricultural Research Service Patent Culture Collection (NRRL), Northern Regional Research Center, 1815 North University Street, Peoria, Illinois 61604. USA. The deposit date was 11 September 1987. The accession number is NRRL B-18251.

B.t.sd. strain MT-38 can be cultured using standard art media and fermentation techniques. For example, see Herrnstadt et al., supra.

The B.t.sd. strain MT-38 spores and crystals may be formulated in a variety of ways. They may be employed as wettable powders, granules or dusts. by mixing with various inert materials, such as inorganic minerals (phyllosilicates. carbonates, sulfates, phosphates. and the like) or botanical materials (powdered corncobs. rice hulls, walnut shells, and the like). The formulations may include spreader-sticker adjuvants, stabilizing agents, other pesticidal additives, or surfactants. Liquid formulations may be aqueous-based or non-aqueous and employed as foams. gels. suspensions. emulsifiable concentrates, or the like. The ingredients may include rheological agents, surfactants, emulsifiers, dispersants, or polymers.

The pesticidal concentration will vary widely depending on the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the coleopteran pest(s), e.g., plants, soil or water, by spraying, dusting, sprinkling or the like. For example, formulated products can be sprayed or applied onto foliage to control phytophagous beetles, or formulated bait granules containing an attractant and B.t.sd. strain MT-38 spores and crystals can be applied to the soil for control of soil-inhabiting Coleoptera. Formulated B.t.sd. strain MT-38 can also be applied as a seed-coating or root treatment for total plant treatment.

Against Diabrotica larvae, which feed on the roots of corn, a bait granule containing cucurbitacin (a phagostimulant for diabroticite beetles derived from cucurbits) or other insect attractants and B.t.sd. strain MT-38 can be formulated. The granules can be planted in the row at planting. Also, a formulated product (wettable powder, etc.) can be sprayed directly on foliage to control susceptible adult beetles.

Alternatively, treated cells of B.t.sd. strain MT-38 can be used to control Coleoptera in comparable formulations, as described above.

Treatment of the substantially intact B.t.sd. strain MT-38 cells can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation.

The cells generally will have enhanced structural stability which will enhance resistance to environmental conditions. Where the protein is in a proform, the method of inactivation should be selected so as not to inhibit processing of the proform to the mature form of the protein by the coleopteran pest. For example, formaldehyde will crosslink proteins and could inhibit processing of the proform of a protein. The method of inactivation or killing retains at least a substantial portion of the bio-availability or bioactivity of the protein toxin.

B.t.sd. strain MT-38 is active against the following coleopteran pests: Dibrotica undecimpunctata - (western spotted cucumber beetle), Diabrotica longicornis (northern corn rootworm), Diabrotica virgitera (western corn rootworm), Diabrotica undecimpunctata howardi (southern corn rootworm), Anthonomus grandis (the cotton boll weevil), Leptinotarsa decemlineata (the Colorado potato beetle), Hypera postica (the alfalfa weevil), Pyrrhalta luteola (elm leaf beetle), Otiorhynchus sulcatus - (black vine weevil), and Tenebrio molitor (yellow mealworm). See Bio/Technology (1986) 4:305-308 for the test procedures. The spectrum of activity for B.t.sd. strain MT-38 is the same as for B.t.sd.

It should be recognized that the process used to prepare B.t.sd. strain MT-38 can be employed to prepare phage-resistant strains of any phage-infected B.t. strains. For example, the process disclosed herein can be used to prepare phage-resistant strains of B. thuringiensis var. tenebrionis described in Krieg et al. Z. ang. Ent. (1983) 96:500-508, if it is infected with bacteriophage.

Following are examples which illustrate procedures, including the best mode, for practicing the invention. These examples should not be construed as limiting. All percentages are by weight and all solvent mixture proportions are by volume unless otherwise noted.

## Example 1: Preparation of B.t.sd. strain MT-38

A lysate of a bacteriophage-contaminated B.t.sd. was prepared from an agar plate that was covered with a lawn of the bacteria that was heavily contaminated with the bacteriophage.

The plate was flooded with sterile saline, and the surface of the plate was gently scraped with a sterile inoculating loop to make a suspension of the cells/bacteriophage. This suspension was pipetted into a sterile centrifuge tube and centrifuged 10 min at 10 K rpm (Sorvall SS-34 rotor, Dupont Instruments, Wilmington, DE) to remove cell debris. The supernatant containing the bacteriophage was pipetted into a sterile tube and refrigerated. Some of this stock was subsequently used to take electron micrographs of the bacteriophage.

To prepare B.t.sd. strain MT-38, first a lawn of the B.t.sd. culture was plated onto nutrient agar plates. The lawn was allowed to dry. A drop of the B.t.sd. phage lysate was then placed on the center of each plate. The drop was allowed to dry and the plates were then incubated at 30°C.

The plates were incubated for 3 days and at that time numerous colonies could be seen growing in the center of the plate where the phage lysate had been plated. Some of these colonies were picked and streaked onto nutrient agar plates.

After overnight incubation, observation of the streaks showed that some had further phage lysis, while others showed no evidence of lysis. The streaks showing no lysis were picked again and streaked onto nutrient agar plates as stock plates. These apparent resistant cultures were tested for resistance by cross-streaking with the phage lysate. A line of the phage lysate was streaked on the plate and allowed to dry. The presumptive resistant cultures were then cross-streaked across the phage line.

Resistant bacterial cultures showed no lysis anywhere in the streak across the phage line after overnight incubating at 30° C.

The presumptive resistant cultures were then tested by plating a lawn of the resistant culture on a nutrient agar plate. After drying, a drop of B.t.sd. phage lysate was added to each plate in the center and allowed to dry. B.t.sd. was used as positive control to show lysis. Resistant cultures showed no lysis in the area where the phage lysate had been placed after incubation at 30° C for 24 hr.

Example 2: Culturing B.t.sd. strain M.T.-38

A subculture of B.t.sd. strain MT-38, NRRL B-18251 can be used to inoculate to following medium known as LB broth:
Tryptone       10 gm
Yeast extract       5 gm
NaCl       5 gm
5N NaOH       0.6 ml
Water 1000.0 ml

As per standard microbiological techniques, the above medium would be sterilized prior to inoculation and the inoculation would be done using aseptic procedures.

A procedure that has produced good results is as follows:

A series of 150 ml Erlenmeyer flasks containing sterile PWYE medium (peptone 5%, yeast extract 0.1%, NaCl 0.5% in 1 liter of water; adjust pH to 7.4) are inoculated from a petri plate culture of B.t.sd. strain MT-38 NRRL B-18251. The flasks are incubated at 30° C on a rotary shaker (200 rpm) overnight. From this starter culture, 300 ml of LB broth in a 2-liter flask is inoculated using 7.5 ml of the starter. The LB-broth flasks are incubated under the same conditions as the starter but are harvested after 4 days.

The above procedure can be readily scaled up to large fermentors by procedures well known in the art.

Upon completion of the fermentation cycle, as evidenced by cell lysis, the B.t.sd. strain MT-38 spores and crystals can be isolated by well-known procedures, e.g., centrifugation. If desired, substantially intact B.t.sd. strain MT-38 cells can be isolated before cell lysis occurs. This isolation can be done by similar well-known procedures.

Example 3.

Upon subjecting a phage-infected B.t. strain to the procedures of Example 1, there is obtained a phage-resistant B.t. strain.

Claims

1. A bacteriophage-resistant strain of Bacillus thuringiensis.

2. A strain according to claim 1, which is active against Coleoptera.

3. A strain according to claim 2, named Bacillus thuringiensis var. san diego.

4. Bacillus thuringiensis var. san diego strain MT-38, NRRL-18251.

5. A strain according to any preceding claim, in the form of intact cells as obtainable by treatment with Lugol's iodine.

6. A process for controlling insect pests, which comprises contacting the pests or their environment, with a bacteriophage-resistant strain according to any of claims 2 to 5.

7. A process according to claim 6, wherein the insect pests are selected from western spotted cucumber beetle, northern corn rootworm, western corn rootworm, southern corn rootworm, cotton boll weevil, Colorado potato beetle, alfalfa weevil, elm leaf beetle, black vine beetle and yellow mealworm.

8. A process according to claim 6 or claim 7, which comprises placing a bait granule incorporating the strain on or in the soil when planting seeds of a plant upon which the insect pests are known to feed.

9. A process according to claim 8, wherein the plant is corn.

10. A composition which comprises a strain according to any of claims 2 to 5 and the beetle stimulant cucurbitacin or another attractant.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS, no. 62040819, Philadelphia, PA., US; C.-Y. SHA et al.: "Studies on phage resistant strains of Bacillus-thuringiensis-var-galleriae 010 and the phages of Bacillus-thuringiensis", & ACTA ENTOMOLogica SINica(CHINA) 1975 vol. 18, no. 3 p273-280 * Abstract * | 1,2 | C 12 N 1/20 A 01 N 63/00 |
| Y | IDEM --- | 3-10 | |
| X | BIOLOGICAL ABSTRACTS, no. 62060362, Philadelphia, PA., US; V.I. ZVENIGORODSKII et al.: "Identification of bacterio phages and study of the properties of phage resistant mutants of Bacillus-thuringiensis-var-galleriae", & BIOLogicheskie NAUKI (MOSCow)(USSR) 1975 vol. 18, no. 5 p92-98 * Abstract * | 1,2 | |
| Y | IDEM --- | 3-10 | TECHNICAL FIELDS SEARCHED (Int. Cl.4) C 12 P C 12 N C 12 R |
| X | JOURNAL OF BACTERIOLOGY, vol. 167, no. 1, July 1986, pages 18-24, American Society for Microbiology, US; A. HEIERSON et al.: "Bacteriophage-resistant mutants of Bacillus thuringiensis with decreased virulence in pupae of Hyalophora cecropia" * Whole article * | 1,2 | |
| Y | IDEM --- -/- | 3-10 | |

The present search report has been drawn up for all claims

| Place of search | Date of completion of the search | Examiner |
|---|---|---|
| THE HAGUE | 10-11-1988 | PULAZZINI A.F.R. |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
........................................................................
& : member of the same patent family, corresponding
  document

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | BIOTECHNOLOGY, vol. 4, no. 4, April 1986, pages 305-308, New York, US; C. HERRNSTADT et al.: "A new strain of Bacillus thuringiensis With activity against coleopteran insects" * Whole article * | 3-10 | |
| Y | EP-A-0 202 739 (MYCOGEN) * Claims 1-4,9,10 * | 10 | |
| Y | EP-A-0 105 608 (ELI LILLY AND CO.) * Page 1, line 3 - page 2, line 15 * | 3-10 | |
| Y | EP-A-0 192 319 (MYCOGEN) * Page 2, line 17 - page 3, line 7; page 6, line 25 - page 9, line 2; page 12, line 10 - page 14, line 15; example 1; claim 1 * | 5-8 | |

TECHNICAL FIELDS
SEARCHED (Int. Cl.4)

The present search report has been drawn up for all claims

| Place of search | Date of completion of the search | Examiner |
|---|---|---|
| THE HAGUE | 10-11-1988 | PULAZZINI A.F.R. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P0401)